# FASCICULE DE BREVET EUROPEEN

(11) **EP 2 082 731 B1**
(45) Date de publication et mention de la délivrance du brevet: **19.02.2014**
(21) Numéro de dépôt: 08163627.6
(22) Date de dépôt: 03.09.2008
(51) Int. Cl.: A61K 8/99, A61Q 19/00, A61K 8/60, A61Q 19/08, A23L 1/30, A61K 8/31, A61K 9/00, A61K 45/06, A61K 9/48, A61K 31/7048, A61K 35/74, A61K 36/062

(54) **Utilisation d'une association d'hespéridine et d'un microorganisme pour agir sur la fonction barrière de la peau**
Verwendung einer Hesperidinverbindung und eines Mikroorganismus zur Beeinflussung der Barrierefunktion der Haut
Use of a combination of hesperidin and a micro-organism to act on the barrier function of the skin

(30) Priorité: 04.09.2007 FR 0757346; 04.09.2007 FR 0757350
(43) Date de publication de la demande: 29.07.2009
(73) Titulaire: L'Oréal, 75008 Paris (FR); Nestec S.A., 1800 Vevey (CH)
(72) Inventeur: Gueniche, Audrey, 92500, RUEIL MALMAISON (FR); Castiel, Isabelle, 06200, NICE (FR)
(74) Mandataire: Le Coupanec, Pascale A.M.P.

(56) Documents cités:
- WO-A-02/28402
- WO-A-03/070260
- WO-A-2005/058255
- WO-A-2006/037922
- DE-A1- 19 806 890
- FR-A- 2 802 088
- FR-A- 2 872 047
- FR-A- 2 889 057

## Description

La présente invention concerne l'utilisation, notamment cosmétique, d'une association, ladite association étant destinée à prévenir une diminution de et/ou renforcer la fonction barrière de la peau et en particulier à prévenir et/ou traiter les désordres associés, et notamment la sécheresse cutanée.

La présente invention concerne également la prévention et/ou le traitement des signes cutanés du vieillissement, notamment de ceux induits ou exacerbés par la pollution.

La peau humaine est constituée de deux compartiments, à savoir un compartiment profond, le derme et un compartiment superficiel, l'épiderme.

Elle constitue une barrière contre les agressions extérieures, notamment chimiques, mécaniques ou infectieuses, et à ce titre un certain nombre de réactions de défense contre les facteurs environnementaux (climat, rayons ultraviolets, tabac, ...) et/ou les xénobiotiques, comme par exemple les micro-organismes, se produisent à son niveau. Cette propriété, appelée fonction barrière, est principalement assurée par la couche la plus superficielle de l'épiderme, à savoir la couche cornée, appelée le *stratum corneum*.

Les cellules constituant l'épiderme (majoritairement les kératinocytes, mais aussi les mélanocytes et les cellules de Langerhans) sont délimitées par une structure lipidique intercellulaire. Chacun de ces types cellulaires contribue par ses fonctions propres au rôle essentiel joué dans l'organisme par la peau. En particulier, les kératinocytes subissent un processus de maturation continu et orienté qui, des kératinocytes se trouvant dans la couche basale de l'épiderme, aboutit à la formation de cornéocytes, qui sont des cellules mortes totalement kératinisées constituées de kératinocytes au stade terminal de leur différenciation.

Au cours de la différenciation, les phospholipides dont le rôle consiste à élaborer la structure fluide des membranes cellulaires des couches vivantes de l'épiderme, sont peu à peu remplacés par un mélange composé en majeure partie d'acides gras, de cholestérol et de sphingolipides (céramides). Ces lipides, qui sont organisés en phases cristal liquide lamellaires spécifiques, forment le ciment intracellulaire du *stratum corneum* et sont essentiels pour les échanges en eau et la fonction barrière de l'épiderme. Ainsi, la structure lamellaire des lipides du domaine lipidique de l'épiderme et les cornéocytes participent à la fonction barrière épidermique.

Il est manifeste que la qualité de la barrière cutanée et des muqueuses est dépendante de mécanismes biologiques endogènes complexes faisant intervenir de nombreux facteurs de croissance, des molécules d'adhésion, des hormones et des enzymes du métabolisme lipidique.

Ainsi une altération de la barrière cutanée peut se produire en présence d'agressions externes de type agents irritants (détergents, acides, bases, oxydants, réducteurs, solvants concentrés, gaz ou fumées toxiques), sollicitations mécaniques (frottements, chocs, abrasion, arrachement de la surface, projection de poussières, de particules, rasage ou épilation), déséquilibres thermiques ou climatiques (froid, sécheresse, radiations), xénobiotiques (micro-organismes indésirable, allergènes) ou d'agressions internes de type stress psychologique.

Cette altération de la barrière cutanée peut notamment se traduire par un inconfort cutané, des phénomènes sensoriels et notamment des phénomènes désagréables ou encore une sécheresse cutanée, qui peut être notamment mesurée par la perte insensible en eau. L'homme peut alors éprouver une sensation d'inconfort cutané qui peut se manifester notamment par des picotements, des tiraillements, une sensation de tension, des échauffements, et/ou des démangeaisons.

Ces sensations d'inconfort cutané sont plus fréquentes dans les zones les plus exposées de l'organisme, à savoir les mains, les pieds, le visage, et le cuir chevelu.

Elles peuvent survenir notamment sur des zones soumises à certains gestes d'hygiène quotidienne ou fréquemment renouvelés tels que le rasage, l'épilation, la détersion par des produits de toilette ou des produits ménagers, l'application d'adhésifs (pansements, patchs, fixation de prothèses) ou dans le cas de gestes sportifs, professionnels ou simplement liés au mode de vie et à l'utilisation de vêtements, d'outils ou d'équipements générant des frottements localisés. Elles peuvent également être amplifiées par le stress psychologique.

Les sensations d'inconfort cutané, les phénomènes sensoriels ou encore la sécheresse cutanée, concernent les personnes ayant tout type de peaux, normal et même gras, et tout particulièrement :
- les personnes à peau dite « fragile » ou « délicate » et vulnérable aux facteurs extérieurs et souvent accompagnée d'érythème et de rosacée, se déséquilibrant rapidement, par exemple, lors de variations de la température ou de l'humidité relative de grande amplitude (cas des peaux de bébé par exemple) ;
- les personnes à peau dite `fragilisée', regroupant notamment :
   - les personnes dont le métabolisme cutané diminue et tout particulièrement dont le film hydro-lipidique protecteur composé de sueur, de sébum et de facteurs d'hydratation naturelle se raréfie, comme c'est le cas pour les personnes âgées de plus de 60 ans et notamment dans le cadre du grand âge (au moins 75 ans). On qualifie ces peaux de « peaux séniles » ;
   - les personnes dont la composition du film hydro-lipidique est modifiée, comme c'est le cas des personnes diabétiques, ou dialysées, ou atteintes de certaines maladies, telle que la xérose vulgaire (d'origine génétique probable et se manifestant en priorité sur le visage, les membres et le dos des mains).

On pourra également parler de personnes à peau « agressée » pour les peaux rasées par exemple.

On recherche donc à prévenir une diminution de et/ou renforcer la fonction barrière cutanée pour :
- prévenir et/ou diminuer les sensations d'inconfort cutané, de picotements, tiraillements, échauffements et démangeaisons, en particulier chez les personnes à peau fragile ou délicate (par exemple les bébés) ; ou les personnes à peau fragilisée (telles que les personnes âgées d'au moins 60 ans et en particulier les personnes d'au moins 75 ans), ou les personnes dont la composition du film hydro-lipidique est modifiée, comme c'est le cas des personnes diabétiques, ou dialysées, ou atteintes de certaines maladies,
- et/ou améliorer la fonction barrière cutanée de peaux atopiques et/ou prolonger les phases de rémission entre des crises aiguës de ce type d'affection.

On cherche également à prévenir une diminution de et/ou renforcer la fonction barrière cutanée pour:
- traiter les états de sécheresse cutanée, les états squameux ; notamment les états pelliculaires ;
- traiter les peaux sèches, notamment les peaux sèches hypo-séborrhéiques ;
- traiter les démangeaisons et/ou tiraillements associés aux peaux sèches ;
- traiter les désordres cutanés liés à un défaut d'excrétion et/ou de sécrétion de sébum ;
- restaurer physiologiquement un état d'hydratation convenable au stratum corneum ;
- traiter les fibres kératiniques sèches ;
- traiter les désordres fonctionnels de l'unité pilo-sébacée ;
- prévenir et/ou réduire les rides liées à une sécheresse cutanée ;
- améliorer le confort des peaux et cuirs chevelus secs ;
- lutter contre l'aspect terne et/ou atone de la peau et/ou des cheveux, conséquences de leur dessèchement.

Pour ce qui concerne plus particulièrement la sécheresse cutanée, elle se manifeste essentiellement par une sensation de tiraillements et/ou de tension. Lorsqu'une peau souffre de sécheresse, celle-ci est aussi rugueuse au toucher et apparaît couverte de squames. Lorsque la sécheresse cutanée est légère, ces squames sont abondantes mais peu visibles à l'oeil nu. Elles sont de moins en moins nombreuses mais de plus en plus visibles à l'oeil nu lorsque ce désordre s'aggrave.

La sécheresse cutanée peut également être associée à une baisse du taux d'hydratation de la barrière cutanée et peut notamment être évaluée par cornéométrie.

L'origine de cette sécheresse cutanée peut être de type constitutionnel ou acquis.

Par ailleurs, au cours du temps, il apparaît différents signes sur la peau, très caractéristiques du vieillissement intrinsèque, se traduisant notamment par une modification de la structure et des fonctions cutanées.

Une autre composante du vieillissement est d'origine exogène (Yaar et Gilchrest, J Invest Dermatol, 1998). En effet, le vieillissement peut-être accéléré par des facteurs environnementaux tels qu'une exposition répétée de la peau à la lumière solaire, et notamment aux rayonnements ultraviolets A et B, ou à la pollution. Ainsi, différents types de produits chimiques, xénobiotiques et particules composent la pollution urbaine. Parmi ces composés, trois grandes catégories de polluants peuvent exercer des effets délétères sur la peau : les gaz, les métaux lourds et les particules, qui sont les résidus de combustion sur lesquelles sont adsorbés de très nombreux composés organiques.

Qui plus est, dans la pollution urbaine, l'exposition concomitante à O₃ et aux UV peut causer un stress oxydatif synergique.

De même, on peut penser qu'il existe une synergie d'action entre l'ozone et les composés organiques issus de la combustion.

Pour des raisons évidentes, on cherche donc de manière permanente à améliorer la résistance de la peau contre les gaz, les métaux lourds, les composés organiques résidus de combustion et leurs effets délétères - maximalisés par les UV - rencontrés notamment dans la pollution urbaine, agissant de manière isolés ou combinés, et de fait pour ralentir les signes d'irritation, de vieillissement et/ou du photovieillissement, notamment induits par l'altération tissulaire induite par lesdits polluants.

Aussi, l'utilisation de substances qui auraient la capacité de protéger les cellules de la peau et la matrice extracellulaire à l'égard des agressions précitées pourrait également réduire les signes et altérations liés au vieillissement et/ou au photovieillissement, notamment ceux induits ou exacerbés par la pollution.

Dans ce cadre, les inventeurs ont découvert que l'association d'hespéridine , d'hespéritine ou de glucoronide d'hespéritine et d'au moins un microorganisme probiotique de l'espèce *Lactobacillus paracasei*, ou de l'une des ses fractions était capable de prévenir une diminution de et/ou de renforcer la fonction barrière de la peau.

En particulier, ils ont mis en évidence qu'une telle association s'avère particulièrement efficace pour prévenir et/ou traiter les états de sécheresse cutanée et en particulier les états de sécheresse cutanée acquise et/ou constitutionnelle.

Une telle constatation repose sur l'observation, par les inventeurs, d'une efficacité de l'association considérée pour traiter la sécheresse des matières kératiniques et troubles associés.

En particulier, les inventeurs ont découvert que l'association considérée selon l'invention pouvait permettre de prévenir et/ou limiter significativement la déshydratation de la peau.

Ainsi, ils ont plus précisément mis en évidence qu'une telle association s'avère particulièrement efficace pour prévenir et/ou traiter les peaux sèches, et encore plus particulièrement les peaux sèches acquises et/ou les peaux sèches constitutionnelles.

Dans le cas d'une sécheresse cutanée acquise, l'intervention de paramètres extérieurs tels que l'exposition aux agents chimiques, à des conditions climatiques difficiles, aux rayons solaires ou bien encore certains traitements thérapeutiques (rétinoïdes, par exemple) est déterminante. Sous ces influences extérieures, la peau peut devenir alors momentanément et localement sèche.

Dans le cas d'une sécheresse cutanée constitutionnelle, on peut distinguer deux catégories : la sécheresse cutanée pathologique et non pathologique.

La sécheresse cutanée constitutionnelle pathologique est essentiellement représentée par la dermatite atopique et les ichthyoses. Elle est quasiment indépendante des conditions extérieures.

La dermatite atopique est décrite comme associée à un déficit dans le métabolisme des lipides du stratum corneum, et notamment des céramides. Cette pathologie se présente sous la forme d'une xérose plus ou moins chronique concernant une grande étendue du corps, associée à des poussées inflammatoires et prurigineuses par plaques.

Les ichthyoses sont des pathologies caractérisées par un déficit génétique affectant le processus de kératinisation à différents stades. Elles se manifestent par une desquamation importante par plaques.

La sécheresse cutanée constitutionnelle non pathologique caractérise des peaux sèches dont la sévérité peut dépendre des facteurs extérieurs déjà évoqués.

L'utilisation de flavonoïdes, dont l'hespéridine, pour augmenter la prolifération cellulaire, et traiter notamment les cicatrices, est déjà connue de WO 03/057210.

WO 2005/058255 décrit par ailleurs l'action de flavanones particuliers, dont l'hespéridine, pour traiter les désordres de la peau et des cheveux, en particulier *via* les propriétés cytoprotectives et anti-inflammatoires de ceux-ci.

FR 2 802 088 et DE 1 980 68 90 décrivent pour leur part les propriétés d'un extrait de citrus dosé en hespéridine pour rétablir ou maintenir l'éclat de la peau ou des cheveux.

Par ailleurs, il est connu de mettre en oeuvre des microorganismes pour le soin et/ou le traitement de matières kératiniques.

Le document WO 02/28402 divulgue la mise en oeuvre de microorganismes probiotiques pour réguler les réactions d'hypersensibilité cutanée, comme les réactions inflammatoires et allergiques, qui relèvent d'un processus inflammatoires.

WO 03/070260, quant à lui, décrit que de tels microorganismes peuvent être utiles à des fins de photoprotection de la peau.

Toutefois, l'association d'hespéridine avec un microorganisme n'y est jamais décrite.

EP 0 774 249 divulgue l'effet d'une combinaison de flavanones spécifiques d'une part et d'une combinaison de flavanones spécifiques avec une céramide particulière d'autre part, sur la différenciation des kératinocytes.

Le document WO 2006/037 922 vise quant à lui des compositions dédiées au traitement des peaux sensibles, mettant en oeuvre une association de deux microorganismes.

Pour ce qui est de FR 2 872 047, il décrit l'association d'un microorganisme probiotique avec un cation minéral divalent.

Enfin, FR 2 889 057 divulgue une composition topique comprenant un microorganisme en association avec un acide gras polyinsaturé et/ou ester d'acide gras polyinsaturé, utile pour le traitement des peaux sensibles.

L'association d'une quantité efficace d'hespéridine, d'hespéritine ou de glucoronide d'hespéritine et d'une quantité efficace d'au moins un microorganisme probiotique de l'espèce *Lactobacillus paracasei*, ou de l'une de ses fractions, n'a ainsi jusqu'à présent jamais été utilisée pour prévenir une diminution de et/ou pour renforcer la fonction barrière de la peau, et en particulier pour prévenir et/ou traiter les désordres associés à la sécheresse cutanée.

De par la prévention d'une diminution et/ou le renforcement de la fonction barrière cutanée induite par l'administration d'une association selon l'invention, toutes les peaux et en particulier les peaux fragiles ou fragilisées (par exemple les peaux de bébé, de personnes d'au moins 60 ans, de préférence d'au moins 75 ans, de personnes diabétiques, ou dialysées), ou souffrant de sécheresse sont mieux protégées des agressions extérieures, chimiques, mécaniques, ou infectieuses.

Les inventeurs ont en particulier démontré sur un modèle de peau reconstruite une amélioration de la fonction barrière de la peau après traitement de ladite peau par l'association selon l'invention.

L'invention concerne ainsi selon un premier de ses aspects, l'utilisation cosmétique d'au moins une quantité efficace d'hespéridine, d'hespéritine ou de glucoronide d'hespéritine en association avec au moins une quantité efficace d'au moins un microorganisme probiotique de l'espèce *Lactobacillus paracasei*, ou l'une de ses fractions, comme agent pour prévenir une diminution de et/ou renforcer la fonction barrière de la peau,et en particulier comme agent pour prévenir et/ou traiter les désordres associées à la sécheresse cutanée.

Elle concerne également l'utilisation cosmétique d'une quantité efficace d'hespéridine, d'hespéritine ou de glucoronide d'hespéritine en association avec au moins une quantité efficace d'au moins un microorganisme probiotique de l'espèce *Lactobacillus paracasei*, ou de l'une de ses fractions, comme agent pour prévenir et/ou traiter les états de sécheresse cutanée.

Elle concerne aussi l'utilisation cosmétique d'une quantité efficace d'hespéridine, d'hespéritine ou de glucoronide d'hespéritine en association avec au moins une quantité efficace d'au moins un microorganisme probiotique de l'espèce *Lactobacillus paracasei*, ou de l'une de ses fractions, comme agent pour prévenir et/ou traiter les peaux sèches, et le cas échéant les désordres associés.

Elle vise aussi l'utilisation d'une quantité efficace d'hespéridine, d'hespéritine ou de glucoronide d'hespéritine en association avec au moins une quantité efficace d'au moins un microorganisme probiotique de l'espèce *Lactobacillus paracasei*, ou de l'une de ses fractions, pour la préparation d'une composition, notamment cosmétique et/ou dermatologique, destinée à prévenir et/ou traiter les états de sècheresse cutanée acquise et/ou constitutionnelle pathologique choisie parmi la dermatite atopique ou l'ichtyose.

L'association considérée selon l'invention permet ainsi d'assurer le maintien de la fonction barrière de la peau à son niveau d'efficacité normale, c'est-à-dire le niveau auquel elle assure sa fonction de protection de l'organisme.

Au sens de l'invention, l'expression « renforcer la fonction barrière de la peau » signifie améliorer la fonction barrière de la peau.

Cette amélioration est en particulier déterminante lorsque la fonction barrière de la peau est altérée et qu'il est nécessaire de la rétablir. Cette altération de la peau peut notamment être due à un état de sécheresse des matières kératiniques et en particulier de sécheresse cutanée.

Elle peut être également avantageuse lorsque l'on souhaite consolider la fonction barrière native de la peau, afin de conférer notamment à l'organisme une meilleure résistance aux agressions extérieures vis-à-vis desquelles il est susceptible d'être exposé.

L'invention concerne également, selon un autre de ses aspects, l'utilisation cosmétique d'au moins une quantité efficace d'hespéridine, d'hespéritine ou de glucoronide d'hespéritine en association avec au moins une quantité efficace d'au moins un microorganisme probiotique de l'espèce *Lactobacillus paracasei*, ou l'une de ses fractions, comme agent pour renforcer la protection de la peau vis-à-vis d'agressions extérieures.

En particulier, ladite association et/ou une composition contenant une telle association peuvent être destinées à prévenir et/ou diminuer un inconfort cutané d'une peau, en particulier induit par un stress exogène d'origine chimique, environnementale, mécanique et/ou un stress endogène, en particulier d'une peau fragile et/ou fragilisée et/ou souffrant de sécheresse, telles que définies précédemment.

L'inconfort cutané peut être notamment caractérisé par des tiraillements, des picotements, des échauffements et/ou des démangeaisons.

Selon un autre mode de réalisation, ladite association et/ou une composition contenant une telle association peuvent être destinées à prévenir une diminution de et/ou renforcer la fonction barrière d'une peau choisie parmi une peau fragile, notamment atopique, une peau fragilisée, une peau agressée et/ou souffrant de sécheresse.

Dans le cadre de l'invention, l'association selon l'invention peut être utilisée pour l'application sur la peau saine, soumise ou pouvant être soumise à l'influence d'agents tels que des agents climatiques et de ce fait susceptible de manifester un inconfort cutané. Dans d'autres cas particuliers, l'association de l'invention peut être appliquée sur la peau lorsqu'elle présente des signes cliniques de déficit de la barrière cutanée, par exemple les peaux atopiques.

L'association selon l'invention ou une composition contenant une telle association selon l'invention peuvent notamment être destinées à prolonger les phases de rémission entre des crises aiguës d'affections dermatologiques, par exemple de type atopie.

Qui plus est, cette prévention d'une diminution de et/ou ce renforcement de la fonction barrière cutanée permet de la rendre plus résistante, notamment aux polluants et aux rayonnements solaires, et, de fait, de protéger les tissus vivants de la peau contre les effets délétères - maximalisés par les UV - des gaz, des métaux lourds et des composés organiques résidus de combustion.

Les inventeurs ont ainsi découvert que l'association considérée selon l'invention pouvait manifester avantageusement une activité protectrice au niveau de la fonction barrière cutanée et permettre ainsi de limiter la pénétration des divers polluants et augmenter la résistance de la peau aux agressions.

Il a ainsi également été constaté qu'une composition comprenant l'association considérée selon l'invention permet de préserver et de protéger la peau contre les effets nocifs de la pollution.

Les inventeurs ont en outre également constaté que l'utilisation de l'association considérée selon l'invention s'avère tout particulièrement efficace, en particulier chez l'adulte, pour le traitement des signes cutanés du vieillissement et/ou du photovieillissement de la peau provoqués par une déficience de la fonction barrière, et, notamment induits ou exacerbés par la pollution, en protégeant la fonction barrière de la peau.

Ainsi la présente invention concerne, selon un autre de ses aspects, une composition cosmétique et/ou dermatologique, notamment utile pour prévenir une diminution de et/ou renforcer la fonction barrière de la peau, et en particulier pour prévenir et/ou traiter les signes cutanés du vieillissement et/ou du photovieillissement, en particulier ceux induits ou exacerbés par la pollution, et/ou des désordres associés à la sècheresse cutanée comprenant, dans un support physiologiquement acceptable, au moins une quantité efficace d'hespéridine, d'hespéritine ou de glucoronide d'hespéritine en association avec au moins une quantité efficace d'au moins un microorganisme de l'espèce *Lactobacillus paracasei*, probiotique ou l'une de ses fractions.

La présente invention concerne également l'utilisation cosmétique d'au moins une quantité efficace d'hespéridine ou de l'un de ses dérivés en association avec au moins une quantité efficace d'au moins un microorganisme, notamment probiotique ou l'une de ses fractions, comme agent pour prévenir et/ou traiter les signes cutanés du vieillissement en particulier induits ou exacerbés par la pollution.

La présente invention concerne également, selon un autre des ses aspects, un procédé de traitement cosmétique de la peau destiné à prévenir une diminution de et/ou renforcer la fonction barrière de la peau, comprenant l'administration d'au moins une quantité efficace d'hespéridine , d'hespéritine ou de glucoronide d'hespéritine en association avec au moins une quantité efficace d'au moins un microorganisme probiotique de l'espèce *Lactobacillus paracasei*, ou l'une de ses fractions.

La présente invention concerne aussi, selon un autre de ses aspects, un procédé de traitement cosmétique pour prévenir et/ou traiter les désordres associés à la sècheresse acquise ou constitutionnelle non pathologique de la peau, comprenant l'administration, par exemple à un sujet présentant une peau sèche, d'au moins une quantité efficace d'hespéridine, d'hespéritine ou de glucoronide d'hespéritine en association avec au moins une quantité efficace d'au moins un microorganisme probiotique de l'espèce *Lactobacillus paracasei*, ou l'une de ses fractions.

La présente invention concerne également, selon un autre de ses aspects, un procédé de traitement cosmétique des signes cutanés du vieillissement notamment des signes induits ou exacerbés par la pollution, comprenant l'administration d'au moins une quantité efficace d'hespéridine, d'hespéritine ou de glucoronide d'hespéritine en association avec au moins une quantité efficace d'au moins un microorganisme probiotique de l'espèce *Lactobacillus paracasei*, ou l'une de ses fractions.

Un procédé selon l'invention peut notamment comprendre au moins une étape consistant à appliquer sur la peau de personnes présentant une peau fragile ou délicate et/ou sur la peau de personnes présentant une peau fragilisée, en particulier la peau de personnes d'au moins 60 ans, voire d'au moins 75 ans, et/ou sur la peau de personnes présentant une peau ou une zone de peau agressée, en particulier la peau rasée du visage ou du corps et/ou une peau souffrant de sécheresse, au moins une quantité efficace de l'association selon l'invention.

Sauf indication contraire, dans le cadre de l'invention, par peau, on entend toute surface cutanée du corps incluant la peau et élargie au cuir chevelu et aux muqueuses et semi-muqueuses.

Au sens de la présente invention, le terme « prévenir » entend le fait de diminuer le risque de survenue d'un phénomène.

Au sens de l'invention, l'expression « prévenir une diminution de la fonction barrière de la peau » signifie prévenir toute altération de ladite fonction barrière en deçà de son niveau d'efficacité naturelle et qui aurait pour conséquence d'initier la manifestation d'un ou plusieurs troubles cutanés tels que définis précédemment.

Par « quantité efficace », on entend au sens de la présente invention une quantité suffisante pour obtenir l'effet attendu.

Par « signes cutanés du vieillissement et/ou du photovieillissement de la peau provoqués par une déficience de la fonction barrière », on entend au sens de la présente invention les altérations du tissu dermique et/ou épidermique, une peau terne, non souple, relâchée par l'altération des fibres élastiques, un aspect flasque de la peau, une perte de tonicité, une altération du microrelief de la peau, de l'ovale du visage, des dyschromies ...

L'association selon l'invention peut être formulée dans des compositions cosmétiques ou dermatologiques.

Selon un mode de réalisation, l'utilisation ou le procédé selon l'invention peut comprendre l'application de l'association selon l'invention par voie topique, ou l'administration orale ou parentérale.

Par voie topique, on entend une administration de l'association selon l'invention ou des compositions qui la comprennent par application sur la peau telle que définie ci-dessus.

Selon un autre mode de réalisation, l'utilisation ou le procédé selon l'invention peut comprendre l'administration de l'association selon l'invention par voie aérienne ou en sous-cutanée.

L'administration en sous cutanée peut notamment être effectuée au moyen d'une seringue.

Comme indiqué précédemment, les voies topiques et orales sont envisageables pour la mise en oeuvre de l'invention.

Les produits topiques agissent néanmoins, par définition, localement sur les zones à traiter, zones sur lesquelles ils peuvent être inégalement répartis, et nécessitent des applications soignées et répétées. Ils peuvent être en outre dans certains cas à l'origine de réactions secondaires cutanées, voire d'inconfort.

Par opposition, la voie orale présente l'avantage d'agir de façon globale sur l'ensemble de la peau et ce dans ses couches profondes (derme, hypoderme), suivant un mode d'administration rapide et peu contraignant. En effet, les métabolites et autres nutriments actifs sont en particulier distribués au sein de la matrice dermique par le biais de la circulation sanguine. La voie orale ou l'administration par patch présentent également l'avantage d'un mode d'administration rapide et peu contraignant.

Selon un mode de réalisation préféré, l'utilisation cosmétique selon l'invention est donc effectuée par voie orale et le procédé selon l'invention comprend l'administration par voie orale de ladite association selon l'invention.

### Microorganismes, et notamment microorganismes probiotiques

Les microorganismes convenant à l'invention sont des microorganismes qui peuvent être administrés sans risques à l'animal ou l'homme.

Le microorganisme est de type probiotique.

Au sens de la présente invention, on entend par « microorganisme probiotique », un microorganisme vivant qui, lorsqu'il est consommé en quantité adéquate, a un effet positif sur la santé de son hôte (« Joint FAO/WHO Expert Consultation on Evaluation of Health and Nutritional Properties of Probiotic in Food Including Powder Milk with Live Lactic Acid Bacteria, 6 octobre 2001 »), et qui peut en particulier améliorer l'équilibre microbien intestinal.

Selon une variante de l'invention, ce microorganisme est mis en oeuvre sous une forme isolée, c'est-à-dire non mélangée à un ou des composé(s) susceptible(s) de lui être associé(s) dans son milieu d'origine.

Au sens de l'invention, le terme « fraction » désigne plus particulièrement un fragment dudit microorganisme doté d'une efficacité pour le traitement des peaux sèches par analogie audit microorganisme entier.

Le microorganisme convenant à l'invention est de l'espèce *Lactobacillus paracasei*.

Ces microorganismes peuvent être formulés à l'état de poudres, c'est-à-dire sous une forme sèche, ou sous forme de suspensions ou de solutions.

Plus particulièrement, il s'agit de microorganismes probiotiques issus du groupe des bactéries lactiques, comme notamment les *Lactobacillus* et/ou les *Bifidobacterium*. A titre illustratif de ces bactéries lactiques, on peut plus particulièrement citer les *Lactobacillus johnsonii, Lactobacillus reuteri, Lactobacillus rhamnosus, Lactobacillus paracasei, Lactobacillus casei* ou *Bifidobacterium bifidum, Bifidobacterium breve, Bifidobacterium longum, Bifidobacterium animalis, Bifidobacterium lactis, Bifidobacterium infantis, Bifidobacterium adolescentis* ou *Bifidobacterium pseudocatenulatum* et leurs mélanges, et de préférence les *Lactobacillus johnsonii, Lactobacillus reuteri, Lactobacillus rhamnosus, Lactobacillus paracasei*, et leurs mélanges.

Selon un mode de réalisation, le microorganisme probiotique est la souche *Lactobacillus paracasei* déposée suivant le traité de Budapest avec l'Institut Pasteur (28 rue du Docteur Roux, F-75024 Paris cedex 15) le 12/01/99 sous la désignation CNCM I-2116.

Selon un mode de réalisation particulier, l'association selon l'invention peut comprendre au moins deux microorganismes ou fractions de ceux-ci, différents.
Le ou les microorganisme(s) peu(ven)t être inclus dans la composition selon l'invention sous une forme vivante, semi-active ou inactivée, morte.
Il(s) peu(ven)t également être inclus sous forme de fractions de composants cellulaires. Le ou le(s) microorganisme(s)ou fraction(s) peu(ven)t également être introduit(s) sous la forme d'une poudre lyophilisée, d'un surnageant de culture et/ou le cas échéant sous une forme concentrée.

D'une manière générale, les compositions selon l'invention peuvent comprendre de 0,00001 à 20 % en poids, en particulier de 0,001 à 20 % en poids et plus particulièrement de 0,01 à 10 % en poids de microorganisme(s), notamment probiotique(s), par rapport au poids total de la composition.

Il peut être avantageux de mettre en oeuvre ces microorganismes sous forme inactivée, voire morte, et plus particulièrement sous forme d'un lysat.

Un tel lysat peut être obtenu à partir de la lyse cellulaire du microorganisme concerné, selon une méthode conventionnelle.

Le(s) microorganisme(s) probiotique(s) sous forme de lysat en suspension désintégré peu(ven)t être formulé(s) dans un support approprié à raison de moins de 20 % en poids, en particulier à raison de 0,0001 à 20 % en poids, et plus particulièrement à raison de 0,01 à 10 % en poids par rapport au poids total dudit support.

Lorsqu'ils sont vivants, les microorganismes et/ou leurs fractions peuvent être formulés dans un support approprié en une quantité équivalente à au moins 10³ ufc/g, en particulier à des doses variant de 10⁵ à 10¹⁵ ufc/g, et plus particulièrement de 10⁷ à 10¹² ufc/g de support.

Aussi, les compositions selon l'invention comprennent généralement de 10³ à 10¹² ufc, en particulier de 10⁵ à 10¹⁰ ufc et plus particulièrement de 10⁷ à 10⁹ ufc de microorganismes vivants notamment probiotiques par gramme de support.

### Hespéridine et ses dérivés

L'hespéridine appartient à la famille des flavanones, qui sont des composés glucosides naturels retrouvés principalement dans les agrumes, c'est-à-dire les fruits du genre *Citrus,* tels que par exemple les oranges, les citrons, les oranges amères ou encore les raisins.

Elles sont présentes majoritairement dans la peau des agrumes mais sont également retrouvées en grandes quantités dans la pulpe, et donc dans le jus des agrumes.

L'hespéridine est un composé glucosylé comprenant un noyau flavanone d'hespéritine (3',5',5-trihydroxy-4'-méthoxyflavanone) auquel est liée de manière covalente une partie glucosidique de rutinose L-rhamnosyl-(α 1→6)-glucose) fixée sur le groupe hydroxyle présent sur le carbone en position 7 de l'hespéritine.

Par hespéridine, on entend ainsi le composé (S)-7[[6-0-(6-deoxy-α-L-mannopyranosyl)-β-D-glucopyranosyl]oxy]-2,3-dihydro-5-hydroxy-2-(3-hydroxy-4-méthoxyphenyl)-4H-1-benzopyran-4-one.

Le composé hespérétine qui est constitué du noyau flavanone non glycosylé de l'hespéridine, a la formule suivante: (S)-2,3-dihydro-5,7-dihydroxy-2-(3-hydroxy-4-méthoxyphényl)-4H-1-benzopyran-4-one;3',5,7-trihydroxy- 4'-méthoxy flavanone;

Le conjugué d'hespérétine et de glucuronide, est retrouvé, avec l' hespérétine, comme produit de la métabolisation de l'hespéridine dans la circulation sanguine.

D'une manière générale, la quantité efficace d'hespéridine ou de l'un de ses dérivés selon l'invention peut être mise en oeuvre à raison de 0,00001 à 20 % en poids, par exemple de 0,001 à 10 % en poids, voire de 5 % à 10 % en poids par rapport au poids total d'une composition en contenant.

De façon générale, dans les procédés et utilisations selon l'invention, la dose d'hespéridine ou de l'un de ses dérivés selon l'invention est mise en oeuvre de manière à administrer, notamment par voie orale, entre 100 mg et 1000 mg, de préférence entre 200 mg et 800 mg, de préférence entre 300 mg et 600 mg et environ 500 mg d'hespéridine ou de l'un de ses dérivés, par personne et par jour.

Avantageusement, une composition selon la présente invention, notamment destinée à une administration orale, contient une quantité d'hespéridine ou de l'un de ses dérivés selon l'invention comprise entre 100 mg et 800 mg, de préférence entre 200 mg et 800 mg, de préférence entre 300 mg et 600 mg et environ 500 mg.

Une telle composition peut notamment se présenter sous la forme d'une gélule ou d'une capsule.

Les compositions selon l'invention peuvent se présenter sous toutes les formes galéniques normalement disponibles pour le mode d'administration retenu.

Le support peut être de nature diverse selon le type de composition considérée.

En ce qui concerne plus particulièrement les compositions destinées à une administration par voie topique externe c'est-à-dire sur la peau, il peut s'agir de solutions aqueuses, hydroalcooliques ou huileuses, de dispersions du type des solutions ou dispersions du type lotion ou sérum, d'émulsions de consistance liquide ou semi-liquide du type lait, de suspensions ou émulsions, du type crème, de gel aqueux ou anhydre, de microémulsions, de microcapsules, de microparticules, ou de dispersions vésiculaires de type ionique et/ou non ionique.

Ces compositions sont préparées selon les méthodes usuelles.

De façon connue, les formes galéniques dédiées à une administration topique peuvent contenir également des adjuvants habituels dans le domaine cosmétique, pharmaceutique et/ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse et/ou dans la phase aqueuse.

Comme matières grasses utilisables dans l'invention, on peut citer les huiles minérales comme par exemple le polyisobutène hydrogéné et l'huile de vaseline, les huiles végétales comme par exemple une fraction liquide du beurre de karité, huile de tournesol et d'amandes d'abricot, les huiles animales comme par exemple le perhydrosqualène, les huiles de synthèse notamment l'huile de Purcellin, le myristate d'isopropyle et le palmitate d'éthyl hexyle, les acides gras insaturés et les huiles fluorées comme par exemple les perfluoropolyéthers. On peut aussi utiliser des alcools gras, des acides gras comme par exemple l'acide stéarique et comme par exemple des cires notamment de paraffine, carnauba et la cire d'abeilles. On peut aussi utiliser des composés siliconés comme les huiles siliconées et par exemple les cyclométhicone et diméthicone, les cires, les résines et les gommes siliconées.

Comme émulsionnants utilisables dans l'invention, on peut citer par exemple le stéarate de glycérol, le polysorbate 60, le mélange alcool cétylstéarylique/alcool cétylstéarylique oxyéthyléné à 33 moles d'oxyde d'éthylène vendu sous la dénomination Sinnowax AO^{®} par la société HENKEL, le mélange de PEG-6/PEG-32/Glycol Stéarate vendu sous la dénomination de Tefose^{®} 63 par société GATTEFOSSE, le PPG-3 myristyl éther, les émulsionnants siliconés tels que le cétyldiméthicone copolyol et le mono- ou tristéarate de sorbitane, le stéarate de PEG-40, le monostéarate de sorbitane oxyéthyléné (20OE).

Comme solvants utilisables dans l'invention, on peut citer les alcools inférieurs, notamment l'éthanol et l'isopropanol, le propylène glycol.

La composition de l'invention peut également contenir de façon avantageuse une eau thermale et/ou minérale, notamment choisie parmi l'eau de Vittel, les eaux du bassin de Vichy et l'eau de la Roche Posay.

Dans le cas d'une utilisation conforme à l'invention par voie orale, on privilégie l'utilisation d'un support ingérable.

Le support ingérable peut être de nature diverse selon le type de composition considérée.

Conviennent ainsi notamment comme supports alimentaires ou pharmaceutiques des comprimés ou des tablettes, des suppléments oraux sous forme sèche et des suppléments oraux sous forme liquide.

Il peut par exemple s'agir de compléments alimentaires, dont la formulation peut être réalisée par les procédés usuels pour produire notamment des dragées, gélules, gels, émulsions, comprimés, capsules et hydrogels permettant une libération contrôlée.

En particulier, l'association selon l'invention peut être incorporée dans toutes autres formes de compléments alimentaires ou d'aliments enrichis, par exemple des barres alimentaires ou des poudres, compactées ou non. Les poudres peuvent être diluées dans de l'eau, du soda, des produits laitiers ou dérivés du soja, ou être incorporées dans des barres alimentaires.

L'association selon l'invention peut être par ailleurs formulée avec les excipients et composants usuels pour de telles compositions orales ou compléments alimentaires, à savoir notamment composants gras et/ou aqueux, agents humectants, épaississants, conservateurs, agents de texture, de saveur et/ou d'enrobage, antioxydants, conservateurs et colorants usuels dans le domaine de l'alimentaire.

Les agents de formulation et excipients pour composition orale, et notamment pour compléments alimentaires, sont connus dans ce domaine et ne font pas ici l'objet d'une description détaillée.

Conviennent notamment comme supports alimentaires ou pharmaceutiques, le lait, le yaourt, le fromage, les laits fermentés, les produits fermentés à base de lait, des glaces, des produits à base de céréales ou des produits à base de céréales fermentées, des poudres à base de lait, des formules pour enfants et nourrissons, des produits alimentaires de type confiserie, chocolat, céréales, des aliments pour animaux en particulier domestiques, des comprimés, gélules ou tablettes, des suspensions de bactéries liquides, des suppléments oraux sous forme sèche et des suppléments oraux sous forme liquide.

Quelque soit le mode d'administration considéré, l'association selon l'invention peut également être avantageusement associée à au moins un autre actif.

A titre d'actifs utilisables, on peut citer, les vitamines A, B3, B5, B6, B8, C, D, E, ou PP, les curcuminoïdes, les caroténoïdes, les composés polyphénols et minéraux, les sucres, les acides aminés, les acides aminés soufrés, les acides gras polyinsaturés 3 et 6, la taurine et les phytostérols.

En particulier, on peut utiliser un complexe anti-oxydant comprenant les vitamines C et E, et au moins un caroténoïde, notamment un caroténoïde choisi parmi le β -carotène, le lycopène, l'astaxanthine, la zéaxanthine et la lutéine, des flavonoïdes telles que les catéchines, des proanthocyanidines, des anthocyanines, des ubiquinones, des extraites de café contenant des polyphénols et/ou des diterpènes, des extraits de chicorés, des extraits de ginkgo biloba, des extraits de raisins riches en proanthocyanidines, des extraits de piment, des extraits de soja, d'autres sources de flavonoïdes possédant des propriétés antioxydantes, des acides gras, des prébiotiques, de la taurine, du resveratrol, des acides aminés du sélénium, des précurseurs de glutathion.

Parmi les flavonoïdes, on choisit de préférence les catéchines et les OPC (oligomères procynidoliques).

Dans les formes galéniques topiques, on peut utiliser plus particulièrement comme actifs hydrophiles les protéines ou les hydrolysats de protéine, les acides aminés, les polyols notamment en C₂ à C₁₀ comme les glycérine, sorbitol, butylène glycol et polyéthylène glycol, l'urée, l'allantoïne, les sucres et les dérivés de sucre, les vitamines hydrosolubles, l'amidon, des extraits bactériens ou végétaux comme ceux d'Aloe Vera.

Pour ce qui est des actifs lipophiles, on peut utiliser le rétinol (vitamine A) et ses dérivés, le tocophérol (vitamine E) et ses dérivés, les céramides, les huiles essentielles et les insaponifiables (tocotriènol, sésamine, gamma oryzanol, phytostérols, squalènes, cires, terpènes).

Comme actifs également susceptibles d'être associés à l'association selon l'invention, convenants à la voie topique mais plus particulièrement adaptés à une formule galénique orale, on peut également considérer tous les ingrédients communément utilisés et/ou autorisés, notamment les agents actifs destinés à la prévention et/ou au traitement des affections cutanées.

A titre illustratif, on peut citer les vitamines, les minéraux, les lipides essentiels, les oligoéléments, les polyphénols, les flavonoïdes, les phytoestrogènes, les antioxydants tels que l'acide lipoïque et le coenzyme Q10, les caroténoïdes, les prébiotiques, les protéines et les acides aminés, les mono et polysaccharides, les amino-sucres, les phytostérols et alcools triterpéniques d'origine végétale.

Il s'agit, en particulier, des vitamines A, C, D, E, PP et du groupe B. Parmi les caroténoïdes, on choisit de préférence, le béta-carotène, le lycopène, la lutéine, la zéazanthine et l'astaxanthine. Les minéraux et oligo-éléments particulièrement mis en oeuvre sont le zinc, le calcium, le magnésium, le cuivre, le fer, l'iode, le manganèse, le sélénium, le chrome (III). Parmi les composés polyphénols, on retient aussi en particulier les polyphénols de raisin, de thé, d'olive, de cacao, de café, de pomme, de myrtille, de sureau, de fraise, de canneberge, et d'oignon. De préférence parmi les phytoestrogènes, on retient les isoflavones sous la forme libre ou glycosylée, telles que la génistéine, la daidzéine, la glycitéine ou encore les lignanes, en particulier ceux du lin et du schizandra chinensis. Les acides aminés ou les peptides et les protéines les contenant, tels que la taurine, la thréonine, la cystéine, le tryptophane, la méthionine. Les lipides appartiennent de préférence au groupe des huiles contenant des acides gras mono et polyinsaturés tels que les acides oléique, linoléique, alpha-linolénique, gamma-linolénique, stearidonique, les acides gras oméga-3 de poisson à longue chaîne tels que l'EPA et le DHA, les acides gras conjugués issus de végétaux ou d'animaux tels que les CLA (Conjugated Linoleic Acid).

Ainsi, en particulier lorsque l'association selon l'invention est destinée à une administration par voie orale, elle peut être associée en outre à au moins un actif nutritionnel choisi parmi le lycopène, la vitamine C, la vitamine E et les composés polyphénols.

L'association selon l'invention peut également être associée à d'autres actifs nutritionnels choisis parmi :
- les actifs nutritionnels anti-âge, tels que les antioxydants alimentaires, les nutriments aux propriétés anti-radicalaires et les cofacteurs des enzymes endogènes antioxydants, les vitamines A, C, E, les caroténoïdes, les xantophyles, les isoflavones, certains minéraux tels que le zinc, le cuivre, le magnésium, le sélénium, l'acide lipoïque, le co-enzyme Q10, la superoxyde dismutase (SOD) ou encore la taurine. Parmi les actifs anti-âges, on peut notamment citer les fractions insaponifiables extraits de lipides d'origine végétale, aloe vera, le collagène marin natif ou hydrolysé, les huiles végétales ou marines riches en acides gras oméga-3, en oméga-6 (y compris l'acide gamma-linolénique),
- les actifs nutritionnels photoprotection tels que : les antioxydants et les antiradicalaires, : les vitamines A, C, E, caroténoïdes, xantophyles, certains minéraux tels que le zinc, le cuivre, le magnésium, le sélénium, la co-enzyme Q10, la superoxyde dismultase (SOD), - les ingrédients nutritionnels présentant des propriétés d'hydratation ou encore immunomodulatrices tels que l'extrait de polypodium leucotomos, les huiles végétales ou marines riches en acides gras oméga-3, en oméga-6, y compris l'acide gamma-linolénique,
- les actifs nutritionnels actifs sur les signes cliniques de la ménopause (par exemple bouffées de chaleur, ...), tels que les isoflavones, les lignanes, la DHEA, les extraits de yam, de sauge, de houblon, le calcium, le magnésium, les hydrolysats de protéines, les huiles végétales ou marines riches en acides gras oméga-3,
- les ingrédients nutritionnels mis en oeuvre dans le domaine de la minceur, tels que les extraits de thé vert, maté, marron d'inde, kola, caféine, théobromine, synéphrine, bromelaïne, éphédra, citrus aurantium, calcium, hoodia, garcinia, chitosan, fibres végétales (cactus, pommes, ananas, ...), fenouil, cassis, reine des près, radis noir.

L'invention se rapporte encore à un procédé de traitement cosmétique pour prévenir une diminution de et/ou renforcer la fonction barrière de la peau, et en particulier pour le soin des peaux âgées, comprenant au moins une étape d'administration de l'association selon l'invention.

Le procédé de traitement cosmétique de l'invention peut être mis en oeuvre notamment en administrant par voie orale et/ou topique au moins une association selon l'invention.

L'administration par voie topique consiste à l'application de compositions cosmétiques et/ou dermatologiques ou associations telles que définies ci-dessus, selon la technique d'utilisation habituelle de ces compositions.

Le procédé cosmétique selon l'invention peut être mis en oeuvre par administration topique, journalière par exemple, de compositions cosmétiques et/ou dermatologiques, ou de l'association selon l'invention qui peut être par exemple formulée sous forme de gels, lotions, émulsions.

L'administration par voie orale consiste à ingérer en une ou plusieurs prises une composition orale telle définie ci-dessus.

Selon une variante, le procédé cosmétique comprend au moins une étape d'administration orale de l'association selon l'invention et au moins une étape d'administration topique de l'association selon l'invention.

Le procédé selon l'invention peut comprendre une administration unique.

Selon un autre mode de réalisation, l'administration est répétée par exemple 2 à 3 fois quotidiennement sur une journée ou plus et généralement sur une durée prolongée d'au moins 4 semaines, voire 4 à 15 semaines, avec le cas échéant une ou plusieurs périodes d'interruption.

Dans la description et dans les exemples suivants, sauf indication contraire, les pourcentages sont des pourcentages en poids et les plages de valeurs libellées sous la forme « entre ... et ... » inclut les bornes inférieure et supérieure précisées.

Les ingrédients sont mélangés, avant leur mise en forme, dans l'ordre et dans des conditions facilement déterminées par l'homme de l'art.

Les exemples et figure ci-après sont présentés à titre illustratif et non limitatif du domaine de l'invention.

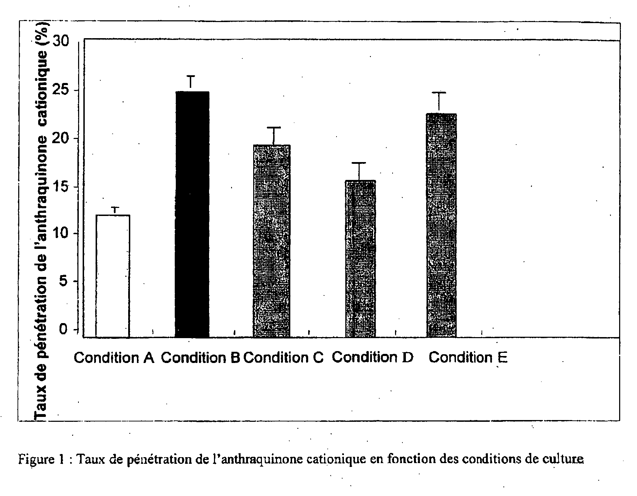

- Figure 1: Taux de pénétration de l'anthraquinone cationique en fonction des conditions de culture (figurée par les lettres A, B, C, D, E).

### Exemple 1 - Évaluation de l'effet d'une association selon l'invention sur la fonction barrière de peaux reconstruites

La transformation d'un agent nutritionnel (appelé ci-après milieu conditionné probiotique) après ingestion est tout d'abord stimulée, en vue d'une utilisation consécutive directement sur un modèle de peau (étape A).

Le milieu des co-cultures stimulées avec le *Lactobacillus paracasei,* issu du compartiment baso-lateral est ensuite prélevé et ses effets en association avec le métabolite le plus important de l'hespéridine, l'hesperitine 7 glucuronide (Hes7Glu) sur la fonction barrière dans le modèle EPISKIN^{®} sont ensuite testés *in vitro* (étape B).

### A - Préparation du milieu conditionné probiotique

Un modèle de barrière intestinale (comprenant des enterocytes humains en lignés (CaCO-2) co-cultivés avec des leucocytes humains dans un système de co-culture cellulaire 'transwell' [Haller D, 2000]) a été développé.

Ce modèle consiste à cultiver séparément :
- une lignée de cellules intestinales CaCO-2 sur des inserts « trans-well » qui sont ensuite placés dans une plaque de 12 puits (Nunc) où les cellules sont cultivées durant 21 jours ; et
- des cellules mono-nucléaires de sang périphérique (leucocytes) humain qui sont purifiées puis resuspendues dans un milieu de culture approprié.

Cette suspension de leucocytes est alors ajoutée dans le compartiment basolatérale des cultures « trans-well » lorsque celles-ci présentent une couche confluente de cellules CaCO-2.

Les co-cultures ainsi établies sont stimulées en ajoutant 1 x 10⁷ CFU/mL de microorganisme probiotique au niveau de la surface apicale de la mono-couche de cellules épithéliales (CaCO-2). Le système est ensuite incubé durant 16h à 37°C/5%CO₂.

En fin d'incubation (16h) le milieu se trouvant dans le compartiment basolatéral est prélevé pour être testé.

Ce modèle de coculture de cellules intestinales et de leucocyte permet de simuler les interactions cellulaires présentent lors de l'ingestion par voie orale d'un ingrédient nutritionnel et de mimer *in vitro*, la situation *in vivo*.

L'interaction des enterocytes activés ou non avec des agents nutritionnels, tels que des probiotiques, agissant au niveau apical entraîne une stimulation des leucocytes sous-jacents et la production de médiateurs (cytokines). Sous l'effet d'une stimulation par des microorganismes probiotiques, ces médiateurs ou autres molécules immunorégulatrices produites au niveau de la muqueuse intestinale sont véhiculés par le sang jusqu'à la peau au niveau de laquelle ils contribuent à son renforcement et/ou à contrebalancer une réaction inflammatoire locale.

### B - Mesure de l'effet de plusieurs agents nutritionnels sur un modèle de peau reconstruite

Les kits Episkin^{®} ont été reçus à J6, puis cultivés pendant la phase proliférative jusqu'à J13 selon cinq conditions

### 1. Condition classique d'Episkin (condition A)

Traités de J6 à J13 avec le milieu de différenciation d'Episkin^{®}

### 2. Contrôle négatif (condition B)

Traités de J6 à J13 avec 30% de milieu de contrôle négatif (milieu conditionné issu de 16h00 de culture CaCO2/PBMC)

### 3. Contrôle positif (condition C)

Traités de J6 à J13 avec 20% de milieu conditionné probiotique.

### 4. Contrôle positif (condition D)

Traités de J6 à J13 avec 20% de milieu conditionné probiotique + Hes7Glu 10µM

### 5. Contrôle positif (condition E)

Traités de J6 à J13 avec Hes7Glu 10µM

Les conditions A, B, C, D et E ont été étudiés sur 6 puits pour chaque lot EPISKIN^{®}, en mesurant la pénétration d'un composé de référence non pénétrant (anthraquinone cationique) formulé dans un milieu simplex. Le taux de pénétration de l'anthraquinone cationique permet de caractériser l'influence des différentes conditions de culture sur la fonction barrière du tissu reconstruit.

Avant l'application, le milieu de culture est retiré et remplacé par 1,5 ml de milieu d'essai Episkin, et les kits sont mis pendant 30 minutes dans une étuve à 37 °C, 5 % CO₂. Une 2^{e} fois, le milieu est retiré et remplacé par du milieu frais, et les kits sont remis pendant 30 minutes en étuve. Enfin, le milieu d'essai Episkin est remplacé par 1,5 ml de PBS+Tween 0,25 % (p/p) et les kits sont placés dans une enceinte thermostatée à 32 °C sous agitation (Certomat).

Le colorant de référence non pénétrant (anthraquinone cationique) a été utilisé. 250 µl d'une formulation simplex tamponnée à pH 7 à une concentration de 1 mM, sont appliqués pendant 4 heures.

Le liquide récepteur (LR) est collecté puis dosé directement en fin d'application, par HPLC. Chaque point est analysé en duplicate.

Lors de la mise au point de la méthode analytique, la spécificité a été vérifiée à partir de blancs LR (liquides récepteurs obtenus après application dans les mêmes conditions de formulation sans colorant).

La concentration est déterminée à l'aide d'une gamme d'étalonnage faite le jour même. Les taux de pénétration sont calculés en effectuant le rapport de la quantité dans le LR sur la quantité appliquée, les résultats suivants ayant fait en outre l'objet d'une étude statistique en utilisant les tests de Wilcoxon.

La condition A utilise le milieu de différenciation d'EPISKIN^{®} comme milieu de culture, qui est optimal pour la différenciation du modèle.

La condition B utilise un milieu dans lequel a été incorporé 30 % de milieu conditionné non stimulé (apparenté à un milieu de culture classique de différenciation EPISKIN^{®} diminué de 30 %). Aussi, la fonction barrière présentée par la condition B a donc été retrouvée plus faible que celle de la condition A.

La figure 1 indique les taux de pénétration du composé de référence anthraquinone cationique dans le liquide récepteur pour chaque condition étudiée.

Il est constaté que les milieux conditionnés issus de la stimulation par 20% *Lactobacillus paracasei* donnent des résultats significativement différents de ceux obtenus dans la condition B contrôle négatif utilisant le milieu non stimulé.

Alors que l'Hes7Glu introduit à une concentration de 10µM n'a pas d'effet significatif sur la fonction barrière, cette même concentration ajoutée à 20 % de milieux conditionnés probiotique permet de retrouver une aussi bonne efficacité de la fonction barrière que celle obtenue avec le milieu de différentiation classique EPISKIN^{®}.

En conséquence, ces résultats montrent clairement une synergie d'activité pour l'Hespéritine 7 glucuronide en association avec te milieu conditionné stimulé avec le probiotique *Lactobacillus paracasei.*

L'introduction de 10M d'Hespéritine 7 glucuronide au milieu de culture supplémentée avec 20 % de milieux conditionnés stimulés par le probiotique *Lucrubacillus paracasei* permet de retrouver une fonction barrière efficace et comparable à celle obtenue dans les conditions de références standard EPISKIN^{®}.

### Exemple 2 : Gel unidose (Hors invention)

| **Principe actif** | **% pds** |
|---|---|
| Hespéridine OBC commercialisée par la société Nutrafur (hespéridine sous forme micronisée pure à 93%) | 10 |
| Lycopène | 10 |
| *Lactobacidlus johnsonii (CNCMI-1225)* | 10¹⁰ cfu |

| **Excipient** | |
|---|---|
| Sirop de sucre | 50 |
| Maltodextrine | 17 |
| Gomme Xanthane | 0,8 |
| Benzoate de sodium | 0,2 |
| Eau | qsp 100 |

On peut prendre une dose de 200 à 400 ml par jour.

### Exemple 3 : Capsule

| | mg/capsule |
|---|---|
| Hespéridine commercialisée par la société SELECTCHEMIE (hespéridine sous forme micronisée pure à 93%) | 10 |
| *Lactobacillus paracasei (CNCM I-2116)* | 10¹⁰ cfu |
| Glycérine | 150 |
| Stéarate de magnésium | 0,02 |
| Arôme naturel | qsp 100 |

On peut prendre une à trois de ces capsules par jour.

### Exemple (Hors invention)

On adjoint à la formulation de l'exemple 2 un complexe vitaminique comportant 60 mg de vitamine C, 100 µg de vitamine E et 6 mg de β-carotène.

### Exemple 5 (Hors invention)

On adjoint à la formulation de l'exemple 2 un complexe vitaminique comportant 100 mg de vitamine C, 100 µg de vitamine E et 6 mg de lycopène par capsule.

### Exemple 6 : Gel unidose

| Principe actif | % pds |
|---|---|
| Hespéridine commercialisée par la société SELECTCHEMIE (hesperidine micronisée pure à 93%) | 10 |
| Lycopène | 10 |
| *Lactobacillus paracasei (CNCM I-2116)* | 10¹⁰ ufc |
| Excipient | |
| Sirop de sucre | 50 |
| Maltodextrine | 17 |
| Gomme Xanthane | 0,8 |
| Benzoate de sodium | 0,2 |
| Eau | qsp 100 |

On peut prendre une dose de 200 à 400 ml par jour.

### Exemple 7 : Capsule

| | mg/capsule |
|---|---|
| Vitamine C | 60 |
| Hespéridine OBC commercialisée par la société Nutrafur (hespéridine micronisée pure à 93%) | 8 |
| *Lactobacillus paracasei (CNCM I-2116)* | 10¹⁰ ufc |
| Glycérine | 150 |
| Stéarate de magnésium | 0,02 |
| Arôme naturel | qsp 100 |

On peut prendre une à trois de ces capsules par jour.

### Exemple 8

On adjoint à la formulation de l'exemple 7 un complexe vitaminique comportant 60 mg de vitamine C, 100 µg de vitamine E et 6 mg de β-carotène.

### Exemple 9

On adjoint à la formulation de l'exemple 7 un complexe vitaminique comportant 100 mg de vitamine C, 100 µg de vitamine E et 6 mg de lycopène par capsule.

### Exemple 10 :

| Crème pour le soin du visage | (% en poids) |
|---|---|
| Hespéritine | 5,00 |
| *Lactobacillus paracasei (CNCM I-2116)* | 10,00 |
| Antioxydant | 0,05 |
| Isopropanol | 40,00 |
| Stéarate de glycérol | 1,00 |
| Alcool cétylstéarylique/alcool cétylstéarylique oxyéthyléné | |
| à 33 moles OE (Sinnowax AO vendu par la société Henkel) | 3,00 |
| Alcool cétylique | 1,00 |
| Diméthicone (DC 200 Fluid vendu par la société Dow Corning) | 1,00 |
| Huile de vaseline | 6,00 |
| Myristate d'isopropyle (Estol IPM 1514 vendu par Unichema) | 3,00 |
| Antioxydant | 0,05 |
| Glycérine | 20,00 |
| Conservateur | 0,30 |
| Eau | qsp 100,00 |

### Exemple 11 : Lotion pour le soin du corps

| | (% en poids) |
|---|---|
| Glucuronide d'hespéritine | 5,00 |
| *Lactobacillus paracasei (CNCM I-2116)* | 10,00 |
| Antioxydant | 0,05 |
| Isopropanol | 40,00 |
| Conservateur | 0,30 |
| Eau | qsp 100,00 |

### Exemple 12 : Lotion pour les mains

| | (% en poids) |
|---|---|
| Hespéritine | 5,00 |
| *Lactobacillus paracasei (CNCM I-2116)* | 10,00 |
| Antioxydant | 0,05 |
| Isopropanol | 40,00 |
| Conservateur | 0,35 |
| Eau | qsp 100,00 |

### Exemple 13 : Gel pour le soin du corps

| | (% en poids) |
|---|---|
| Glucuronide d'hespéritine | 5,00 |
| *Lactobacillus paracasei (CNCM I-2116)* | 10,00 |
| Antioxydant | 0,05 |
| Vitamine C | 2,50 |
| Antioxydant | 0,05 |
| Isopropanol | 40,00 |
| Conservateur | 0,30 |
| Eau | qsp 100,00 |

### Exemple 14 : Capsule

| | mg/capsule |
|---|---|
| Vitamine C | 60 |
| Hespéridine OBC commercialisée par la société Nutrafur (hespéridine micronisée pure à 93%) | 500 |
| *Lactobacillus paracasei (CNCM I-2116)* | 10⁹ ufc |
| Glycérine | 150 |
| Stéarate de magnésium | 0,02 |
| Arôme naturel | qsp 1000 |

On peut prendre une à trois de ces capsules par jour.

### Exemple 15 : Capsule

| | mg/capsule |
|---|---|
| Hespéridine commercialisée par la société SELECTCHEMIE (hespéridine sous forme micronisée pure à 93%) | 500 |
| *Lactobacillus paracasei (CNCM I-2116)* | 10⁹ cfu |
| Glycérine | 150 |
| Stéarate de magnésium | 0,02 |
| Arôme naturel | qsp 1000 |

On peut prendre une à trois de ces capsules par jour.

## Revendications

1. Utilisation cosmétique non thérapeutique d'au moins une quantité efficace d'hespéridine, d'hespéritine ou de glucuronide d'hespéritine, en association avec au moins une quantité efficace d'au moins un microorganisme probiotique de l'espèce *Laccobacillus paracasei,* ou l'une de ses fractions, comme agent pour prévenir une diminution de et/ou renforcer la fonction barrière de la peau

2. Utilisation cosmétique non thérapeutique pour application sur une peau saine, d'au moins une quantité efficace d'hespéridine, d'hespéritine ou de glucuronide d'hespéritine, en association avec au moins une quantité efficace d'au moins un microorganisme probiotique de l'espéce *Lactobacillus paracasei*, ou l'une de ses fractions, comme agent pour prévenir et/ou diminuer l'inconfort cutané d'une peau saine soumise à l'influence d'agents climatiques.

3. Utilisation selon la revendication 1, **caractérisée en ce que** ladite association est destinée à prévenir et/ou diminuer l'inconfort cutané d'une peau, en particulier induit par un stress exogène d'origine chimique, environnementale, mécanique et/ou un stress endogène.

4. Utilisation selon la revendication 2 ou 3, **caractérisée en ce que** l'inconfort cutané est **caractérisé par** des tiraillements, des picotements, une sensation de tensions, des échauffements et/ou des démangeaisons.

5. Utilisation selon l'une quelconque des revendications précédentes, **caractérisée en ce que** ladite association est destinée à prévenir une diminution de et/ou renforcer la fonction barrière d'une peau choisie parmi une peau fragile, notamment atopique, une peau fragilisée, une peau agressée et/ou souffrant de sécheresse.

6. Utilisation cosmétique non thérapeutique d'une quantité efficace d'hespéridine, d'hespéritine ou de glucuronide d'hespéritine, en association avec au moins une quantité efficace d'au moins un microorganisme probiotique de l'espèc *Lactobacillus paracasei*, ou de l'une de ses fractions, comme agent pour prévenir et/ou traiter une peau sèche acquise ou constitutionnelle non pathologique.

7. Utilisation d'une quantité efficace d'hespéridine, d'hespéritine ou de glucuronide d'hespéritine, en association avec au moins une quantité efficace d'au moins un microorganisme, probiotique de l'espèce *Lactobacillus paracasei*, ou de l'une de ses fractions, pour la préparation d'une composition dermatologique destinée à prévenir et/ou traiter une peau sèche constitutionnelle pathologique choisie parmi la dermatite atopique ou l'ichtyose.

8. Utilisation cosmétique non thérapeutique d'au moins une quantité efficace d'hespéridine, d'hespéritine ou de glucuronide d'hespéritine, en association avec au moins une quantité efficace d'au moins un microorganisme probiotique de l'espéce *Lactobacillus paracasei*, ou l'une de ses fractions, comme agent pour prévenir et/ou traiter les signes cutanés du vieillissement.

9. Utilisation cosmétique non thérapeutique d'au moins une quantité efficace d'hespéridine, d'hespéritine ou de glucuronide d'hespéritine, en association avec au moins une quantité efficace d'au moins un microorganisme probiotique de l'espèce *Lactobacillus paracasei*, ou l'une de ses fractions pour une activité protectrice au niveau de la fonction barrière cutanée pour limiter la pénétration de polluants et augmenter la résistance de la peau aux agressions.

10. Utilisation selon l'une quelconque des revendications précédentes, **caractérisée en ce que** le microorganisme est la souche *Lactobacillus paracasei* déposée le 12/01/99 sous la désignation CNCM I-2116.

11. Utilisation selon l'une quelconque des revendications précédentes, dans laquelle ledit microorganisme est mis en oeuvre à raison de 0,00001 à 20 % en poids, en particulier de 0,001 à 20 % en poids et plus particulièrement de 0,01 à 10 % en poids par rapport au poids total d'une composition en contenant.

12. Utilisation selon l'une quelconque des revendications précédentes, dans laquelle la quantité efficace d'hespéridine, d'hespéritine ou de glucuronide d'hespéritine, est mise en oeuvre à raison de 0,00001 à 20% en poids, par exemple de 0,001 à 10 % en poids par rapport au poids total d'une composition en contenant.

13. Utilisation selon ses revendications 1 et 5 à 8, comprenant l'administration de ladite association par voie topique ou orale.

14. Utilisation selon l'une quelconque des revendications 1 à 12, **caractérisée en ce que** la dose d'hespéridine, d'hespéritine ou de glucuronide d'hespéritine, est mise en oeuvre de manière à administrer entre 100 mg et 1000 mg, de préférence entre 200 mg et 800 mg, de préférence entre 300 mg et 600 mg et environ 500 mg d'hespéridine, d'hespéritine ou de glucuronide d'hespéritine, par personne et par jour.

15. Composition cosmétique et/ou dermatologique, notamment utile pour prévenir une diminution de et/ou renforcer la fonction barrière de la peau, et en particulier pour prévenir et/ou traiter les signes cutanés du vieillissement et/ou du photovieillissement et/ou les désordres associés à la sécheresse cutanée comprenant, dans un support physiologiquement acceptable, au moins une quantité efficace d'hespéridine, d'hespéritine ou de glucuronide d'hespéritine, en association avec au moins une quantité efficace d'au moins un microorganisme probiotique de l'espèce *Lactobacillus paracasei* ou l'une de ses fractions

16. Procédé de traitement cosmétique non thérapeutique de la peau destiné à prévenir une diminution de et/ou renforcer la fonction barrière de la peau, comprenant l'administration d'au moins une quantité efficace d'hespéridine, d'hespéritine ou de glucuronide d'hespéritine, en association avec au moins une quantité efficace d'au moins un microorganisme probiotique de l'espéce *lactobacillus parasei*, ou l'une de ses fractions

17. Procédé de traitement cosmétique non thérapeutique des signes cutanés du vieillissement induits ou exacerbés par la pollution, comprenant l'administration d'au moins une quantité efficace d'hespéridine, d'hespéritine ou de glucuronide d'hespéritine, en association avec au moins une quantité efficace d'au moins un microorganisme probiotique de l'espèce *Lactobacillus paracasei*, ou l'une de ses fractions.

18. Procédé de traitement cosmétique non thérapeutique pour prévenir et/ou traiter une peau sèche acquise ou constitutionnelle non pathologique, comprenant l'administration, d'au moms une quantité efficace d'hespéridine, d'hespéritine ou de glucuronide d'hespéritine, en association avec au moins une quantité efficace d'au moins un microorgamsme probiotique de l'espèce *Lactobacillus paracasei,* ou l'une de ses fractions.

## Patentansprüche

1. Nichttherapeutische kosmetische Verwendung wenigstens einer wirksamen Menge von Hesperidin, Hesperitin oder Hesperitin-Glucuronid, eines seiner Derivate in Zuordnung zu wenigstens einer wirksamen Menge wenigstens eines probiotischen Mikroorganismus der Gattung *Lactobacillus Paracasei* oder einer ihrer Fraktionen als Wirkstoff, um eine Verringerung der Barrierenfunktion der Haut zu verhindern und/oder um sie zu verstärken.

2. Nichttherapeutische kosmetische Verwendung für die Anwendung auf gesunder Haut wenigstens einer wirksamen Menge von Hesperidin, Hesperitin oder Hesperitin-Glucuronid in Zuordnung zu wenigstens einer wirksamen Menge wenigstens eines probiotischen Mikroorganismus der Gattung *Lacfobacillus Paracasei* oder einer ihrer Fraktionen als Wirkstoff, um Hautprobleme einer gesunden Haut, die dem Einfluss klimatischer Wirkstoffe unterliegt, zu verhindern und/oder zu verringern.

3. Verwendung nach Anspruch 1, **dadurch gekennzeichnet, dass** die Zuordnung dazu bestimmt ist, ein Hautproblem einer Haut zu verhindern und/oder zu verringern, das insbesondere durch exogene Beanspruchung mit chemischem Ursprung, Umweltursprung oder mechanischem Ursprung und/oder durch eine endogene Beanspruchung eingeführt wird.

4. Verwendung nach Anspruch 2 oder 3, **dadurch gekennzeichnet, dass** das Hautproblem **gekennzeichnet ist durch** ziehende Schmerzen, Kribbeln, eine Empfindung von Spannungen, Erwärmung und/oder Juckreize.

5. Verwendung nach einem der vorhergehenden Ansprüche, **dadurch gekennzeichnet, dass** die Zuordnung dazu bestimmt ist, eine Abnahme der Barrierenfunktion einer Haut zu verringern und/oder um sie zu verstärken, wobei die Haut unter schwacher, insbesondere atopischer Haut, empfindlicher Haut, angegriffener und/oder unter Trockenheit leidender Haut gewählt ist.

6. Nichttherapeutische kosmetische Verwendung einer wirksamen Menge von Hesperidin, Hesperitin oder Hesperitin-Glucuronid in Zuordnung zu wenigstens einer wirksamen Menge wenigstens eines probiotischen Mikroorganismus der Gattung *Lactobacillus Paracasei* oder eines ihrer Fraktionen als Wirkstoff, um trockene Haut, die sicher nicht pathologisch ist und sicher in einem nicht pathologischen Zustand ist, zu verhindern und/oder zu behandeln.

7. Verwendung einer wirksamen Menge von Hesperidin, Hesperitin oder Hesperitin-Glucuronid in Zuordnung zu wenigstens einer wirksamen Menge eines probiotischen Mikroorganismus der Gattung *Lactobacillus Paracasei* oder einer ihrer Fraktionen für die Zubereitung einer dermatologischen Zusammensetzung, die dazu bestimmt ist, eine in einem pathologischen Zustand befindliche trockene Haut, die unter Neurodermatitis oder lchthyose gewählt ist, zu verhindern und/oder zu behandeln.

8. Nichttherapeutische kosmetische Verwendung wenigstens einer wirksamen Menge von Hesperidin, Hesperitin oder Hesperitin-Glucuronid in Zuordnung zu wenigstens einer wirksamen Menge wenigstens eines probiotischen Mikroorganismus der Gattung *Lactobacillus Paracasei* oder einer ihrer Fraktionen als Wirkstoff, um Hautalterungsanzeichen zu verhindern und/oder zu behandeln.

9. Nichttherapeutische kosmetische Verwendung wenigstens einer wirksamen Menge von Hesperidin, Hesperitin oder Hesperitin-Glucuronid in Zuordnung zu wenigstens einer wirksamen Menge wenigstens eines probiotischen Mikroorganismus der Gattung *Lactobacillus Paracasei* oder einer ihrer Fraktionen für eine schützende Aktivität auf Höhe der Hautbarrierenfunktion, um ein Eindringen von Verschmutzungen zu begrenzen und um den Widerstand der Haut gegenüber Angriffen zu erhöhen.

10. Verwendung nach einem der vorhergehenden Ansprüche, **dadurch gekennzeichnet, dass** der Mikroorganismus der Stamm *Lactobacillus Paracasei* ist, der am 12.01.99 unter der Bezeichnung CNCM I-2116 hinterlegt worden ist, ist.

11. Verwendung nach einem der vorhergehenden Ansprüche, wobei der Mikroorganismus in einem Anteil im Bereich von 0,00001 bis 20 Gewichts-%, insbesondere von 0,0001 bis 20 Gewichts-% und noch spezieller im Bereich von 0,001 bis 10 Gewichts-% in Bezug auf das Gesamtgewicht einer ihn enthaltenden Zusammensetzung eingesetzt wird.

12. Verwendung nach einem der vorhergehenden Ansprüche, wobei die wirksame Menge von Hesperidin, Hesperitin oder Hesperitin-Glucuronid in einem Anteil im Bereich von 0,00001 bis 20 Gewichts-%, beispielsweise im Bereich von 0,001 bis 10 Gewichts-%, im Verhältnis zum Gesamtgewicht einer sie enthaltenden Zusammensetzung eingesetzt wird.

13. Verwendung nach den Ansprüchen 5 bis 8, die die Verabreichung der Zuordnung auf topischem oder oralem Weg umfasst.

14. Verwendung nach einem der Ansprüche 1 bis 12, **dadurch gekennzeichnet, dass** die Dosis von Hesperidin, Hesperitin oder Hesperitin-Glucuronid in der Weise eingesetzt wird, dass Hesperidin, Hesperitin oder Hesperitin-Glucuronid im Bereich von 100 mg bis 1000 mg, vorzugsweise im Bereich von 200 mg bis 800 mg, noch stärker bevorzugt im Bereich von 300 mg bis 600 mg und speziell etwa 500 mg pro Person und pro Tag verabreicht wird.

15. Kosmetische und/oder dermatologische Zusammensetzung, die insbesondere nützlich ist, um eine Abnahme der Barrierenfunktion der Haut zu verhindern und/oder um sie zu verstärken, um insbesondere Alterungsanzeichen und/oder Lichtalterungsanzeichen der Haut und/oder Krankheiten in Verbindung mit Hauttrockenheit zu verhindern und/oder zu behandeln, die in einem physiologisch akzeptablen Träger wenigstens eine wirksame Menge von Hesperidin, Hesperitin oder Hesperitin-Glucuronid in Zuordnung zu wenigstens einer wirksamen Menge wenigstens eines probiotischen Mikroorganismus *Lactobacillus Paracasei* oder einer ihrer Fraktionen umfasst.

16. Nichttherapeutische kosmetisches Behandlungsverfahren der Haut, das dazu bestimmt ist, eine Abnahme der Barrierenfunktion der Haut zu verhindern und/oder um sie zu verstärken, das umfasst: Verabreichen wenigstens einer wirksamen Menge von Hesperidin, Hesperitin oder Hesperitin-Glucuronid in Zuordnung zu wenigstens einer wirksamen Menge wenigstens eines probiotischen Mikroorganismus der Gattung *Lactobacillus Paracasei* oder einer ihrer Fraktionen.

17. Nichttherapeutische kosmetisches Behandlungsverfahren von Hautalterungsanzeichen, die durch Verschmutzung eingeführt oder verschlimmert werden, das umfasst: Verabreichen wenigstens einer wirksamen Menge von Hesperidin, Hesperitin oder Hesperitin-Glucuronid in Zuordnung zu wenigstens einer wirksamen Menge wenigstens eines probiotischen Mikroorganismus der Gattung *Lactobacillus Paracasei* oder einer ihrer Fraktionen.

18. Nichttherapeutische kosmetisches Behandlungsverfahren, um trockene Haut, die sicher nicht pathologisch ist und sicher in einem nicht pathologischen Zustand ist, zu verhindern und/oder zu behandeln, das umfasst: Verabreichen wenigstens einer wirksamen Menge von Hesperidin, Hesperitin oder Hesperitin-Glucuronid in Zuordnung zu wenigstens einer wirksamen Menge wenigstens eines probiotischen Mikroorganismus der Gattung *Lactobacillus Paracasei* oder einer ihrer Fraktionen.

## Claims

1. The non-therapeutic cosmetic use of at least an effective quantity of hesperidin, hesperitin or hesperitin glucuronide in association with an effective quantity of at least one probiotic microorganism of the species *Lactobacillus paracasei* or one of the fractions thereof as agent to prevent a reduction in and/or to reinforce the barrier function of the skin.

2. The non-therapeutic cosmetic use for application to healthy skin of at least an effective quantity of hesperidin, hesperitin or hesperitin glucuronide in association with at least an effective quantity of at least one probiotic microorganism of the species *Lactobacillus paracasei* or one of the fractions thereof, as agent to prevent and/or reduce skin discomfort of healthy skin subjected to the influence of climate-related agents.

3. The use according to claim 1, **characterized in that** the said association is intended to prevent and/or reduce skin discomfort induced in particular by exogenous stress of chemical, environmental, mechanical origin and/or endogenous stress.

4. The use according to claim 2 or 3, **characterized in that** skin discomfort is **characterized by** tightness, tingling, feeling of tautness, hotness and/or itching.

5. The use according to any of the preceding claims, **characterized in that** the said association is intended to prevent a reduction in and/or to reinforce the barrier function of skin chosen from among fragile skin, atopic in particular, weakened skin, skin subjected to harsh conditions and/or suffering from dryness.

6. The non-therapeutic cosmetic use of an effective quantity of hesperidin, hesperitin or hesperitin glucuronide in association with at least an effective quantity of at least one probiotic microorganisms of the species *Lactobacillus paracasei* or one of the fractions thereof as agent to prevent and/or treat acquired or non-pathological constitutional dry skin.

7. The use of an effective quantity of hesperidin, hesperitin or hesperitin glucuronide in association with at least an effective quantity of at least one probiotic microorganism of the species *Lactibacillus paracasei* or one of the fractions thereof to prepare a dermatological composition intended to prevent and/or treat pathological constitutional dry skin chosen from among atopic dermatitis or ichthyosis.

8. The non-therapeutic cosmetic use of at least an effective quantity of hesperidin, hesperitin or hesperitin glucuronide in association with at least an effective quantity of at least one probiotic microorganism of the species *Lactobacillus paracasei* or one of the fractions thereof as agent to prevent and/or treat signs of skin ageing.

9. The non-therapeutic cosmetic use of at least an effective quantity of hesperidin, hesperitin or hesperitin glucuronide in association with at least an effective quantity of at least one probiotic microorganism of the species *Lactobacillus paracasei* or one of the fractions thereof for protective action at the skin barrier function to limit entry of pollutants and increase skin resistance to attack.

10. The use according to any of the preceding claims, **characterized in that** the microorganism is the strain *Lactibacillus paracasei* deposited on 12 January 1999 under number CNCM I-2116.

11. The use according to any of the preceding claims wherein the said microorganism is used to the proportion of 0.00001 to 20 % by weight, in particular 0.001 to 20 % by weight and more particularly from 0.01 to 10 % by weight relative to the total weight of a composition in which it is contained.

12. The use according to any of the preceding claims wherein the effective quantity of hesperidin, hesperitin or hesperitin glucuronide is used at a proportion of 0.00001 to 20 % by weight, for example 0.001 to 10 % by weight relative to the total weight of a composition in which it is contained.

13. The use according to claims 1 and 5 to 8 comprising the administering of said association via topical or oral route.

14. The use according to any of claims 1 to 12, **characterized in that** the dose of hesperidin, hesperitin or hesperitin glucuronide is used so as to administer between 100 mg and 1000 mg, preferably between 200 mg and 800 mg, preferably between 300 mg and 600 mg and about 500 mg of hesperidin, hesperitin or hesperitin glucuronide per person per day.

15. A cosmetic and/or dermatological composition useful in particular to prevent a reduction in and/or to reinforce the skin barrier function, and in particular to prevent and/or treat signs of skin ageing and/or photoageing and/or disorders associated with skin dryness containing, in a physiologically acceptable carrier, at least an effective quantity of hesperidin, hesperitin or hesperitin glucuronide in association with at least an effective quantity of at least one probiotic microorganism of the species *Lactobacillus paracasei* or one of the fractions thereof.

16. A non-therapeutic cosmetic method for treating skin intended to prevent a reduction in and/or to reinforce the skin barrier function, comprising the administering of at least an effective quantity of hesperidin, hesperitin or hesperitin glucuronide in association with at least an effective quantity of at least one probiotic microorganism of the species *Lactobacillus paracasei* or one of the fractions thereof.

17. A non-therapeutic cosmetic method for treating signs of skin ageing induced or aggravated by pollution, comprising the administering of at least an effective quantity of hesperidin, hesperitin or hesperitin glucuronide in association with at least an effective quantity of at least one probiotic microorganism of the species *Lactobacillus paracasei* or one of the fractions thereof.

18. A non-therapeutic cosmetic treatment method to prevent and/or treat acquired or non-pathological constitutional dry skin comprising the administering of at least an effective quantity of hesperidin, hesperitin or hesperitin glucuronide in association with at least an effective quantity of at least one probiotic microorganism of the species *Lactobacillus paracasei* or one of the fractions thereof.
